(19) Europäisches Patentamt / European Patent Office / Office européen des brevets

(11) **EP 3 682 899 A1**

(12) **EUROPEAN PATENT APPLICATION**

(43) Date of publication:
**22.07.2020 Bulletin 2020/30**

(21) Application number: **19176088.3**

(22) Date of filing: **23.05.2019**

(51) Int Cl.:
*A61K 41/00* (2020.01)   *A61K 47/64* (2017.01)
*A61K 47/69* (2017.01)   *A61P 35/00* (2006.01)
*C07K 7/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.01.2019  CN 201910034439**
**18.01.2019  CN 201910047595**

(71) Applicant: **Tianjin University**
**Tianjin 300072 (CN)**

(72) Inventor: **LIU, Zhe**
**Tianjin City, Tianjin (CN)**

(74) Representative: **Kransell & Wennborg KB**
**P.O. Box 2096**
**403 12 Göteborg (SE)**

(54) **COMPOUND AMPHIPHILIC PEPTIDE NANOMICELLE, PREPARATION AND USE THEREOF**

(57)   Disclosed is a composite amphiphilic peptide nanomicelle, the preparation method and application, as to a novel integrin $\alpha_v\beta_3$-targeted amphiphilic peptide nanomicelles, the applications in fluorescence imaging, photodynamic therapy, sonodynamic therapy and combined treatments. Based on the prominent properties of integrin $\alpha_v\beta_3$-targeted amphiphilic peptide nanomicelles such as great biocompability, fluorescence imaging of encapsulated materials, photodynamic therapy and photothermal therapy, it is promising to be widely used in the field of labeling and tracing in vivo, biomedical imaging, early detection and treatment for tumor. It has good economic and social benefits in terms of life health and personalized medicine.

▲   Fluorescence Substances (ICG, RB, MB, DOX)

●━━━   RGD Sequence-included Amphiphilic Peptide

Fig. 1

EP 3 682 899 A1

**Description**

CROSS-REFERENCE TO RELATED APPLICATIONS

The present application claims priorities to Chinese Patent Application No. 201910034439.9, filed on January 15, 2019 with China National Intellectual Property Administration (CNIPA) and entitled "Compound amphiphilic peptide nanomicelle, preparation and use thereof', and Chinese Patent Application No. 201910047595.9 filed on January 18, 2019 with China National Intellectual Property Administration (CNIPA) and entitled "Nanomicelle for multi-mode therapy for nasopharyngeal carcinoma, preparation method and use thereof', the contents of which are herein incorporated by reference in their entirety.

TECHNICAL FIELD

The present application relates to a novel amphiphilic peptide self-assembling micelle and a preparation process, and a biomedical application use as a potential photodynamic and sonodynamic therapeutic agent.

BACKGROUND

Malignant tumors such as breast cancers, nasopharyngeal carcinoma (NPC), vertical cancers and melanomas are commonly diagnosed cancers all around the world. Taking NPC as an example, it is common in Guangdong Province and Guangxi Zhuang Autonomous region and harmful to the health of the people in the high incidence area. Radiation therapy is the first choice for the treatment of nasopharyngeal carcinoma without distant metastasis, which has great control efficiency for early nasopharyngeal carcinoma. For advanced nasopharyngeal carcinoma, the mode of radiotherapy combined with chemotherapy is often used in clinical. However, the acute poisonous effect associated with radiotherapy and chemotherapy is harmful to human body, and some individuals with weak tolerance are not suitable to bear the side effects, thus bringing new difficulties to the implementation of the treatment plan. In addition, distant metastasis is another major cause of failure in the treatment of nasopharyngeal cancer.

Melanoma is a clinically common skin mucosa and pigmented malignant tumor, and it is also one of the fastest growing malignant tumors with an annual growth rate of 3%-5%. Although the incidence of melanoma is relatively low in China, it has increased multiply in recent years, with about 20,000 new cases per year. The only evidence for the etiology of melanoma is related to excessive exposure to UV radiation, but the primary lesions of melanoma patients in Asia (including China) and Africa are mostly located in the heel, palm, finger toe and underarm. The cause of the disease is still unclear. In recent years, with the deepening of the research on the relationship between molecular biological characteristics, clinical histological features and genetic variation of melanoma, it has been found that specific types are associated with specific genetic variations. The diagnosis of melanoma is mainly based on typical clinical manifestations and physical signs. Pathological examination is the gold standard for diagnosis and staging. Immunohistochemical staining is the main auxiliary method for identifying melanoma. S-100, HMB-45 and vimentin are more specific indicators for the diagnosis of melanoma. The treatment of melanoma is mainly surgical treatment, postoperative adjuvant treatment, radiotherapy and systemic treatment.

In addition, for cervical cancer, there are 510,000 cases per year and 288,000 deaths worldwide, which is second only to the incidence of breast cancer, and nearly 3/4 cases occur in developing countries. Some patients who are elderly, have poor economic status, and do not participate in cervical cancer smear screening can progress and die of cancer. About 80% of cervical cancer histology is squamous cell carcinoma, and 15% is adenocarcinoma. Although the prognosis of adenocarcinoma is not ideal, there is no data to prove that it is different from treatment methods for other cervical cancer. Pap smear is the most important method for the detection of early cervical cancer. Patients with suspected pre-invasive lesions may be examined by colposcopy, cervical canal curettage, etc. Cystoscopy or rectal biopsy is required for suspected bladder or rectal lesions. Histology confirmed that stage IB1 or above should be examined by pelvic and abdominal CT or MRI so as to determine clinical stage, which is of great significance for the development of follow-up treatment. The treatment of cervical cancer mainly depends on surgical treatment, as well as radiotherapy and chemotherapy.

Therefore, melanoma and cervical cancer have become one of the serious diseases endangering the health of our people. How early detection and effective treatment become the focus of current research.

Fluorescence imaging is widely used in the field of biological detection and medical imaging, which is a common optical imaging technology. The agent for fluorescence imaging has the advantages of high penetration, sensitivity and selectivity. However, traditional organic dyes also have some fatal defects, such as unstable properties, easy to be photo-bleached, and cannot be used for a long time. Therefore, how to overcome the defects in the use of fluorescent dyes has become the focus of current research.

Photodynamic therapy (PDT) and sonodynamic therapy (SDT) are new methods approved by the FDA in recent years to use light and ultrasound for treating disease. PDT refers to the decomposition of photosensitizer to release reactive oxygen species (ROS) to kill tumor cells under the action of light. SDT means that the sonosensitizer is excited to release ROS that can kill tumor cells under ultrasound. SDT/PDT only require laser or ultrasound without harm to human body, which are non-invasive treatment methods. It greatly alleviates the pain caused by surgery, chemotherapy and radiation therapy, especially for the elderly and frail patients who can no longer afford surgery. In addition, treatment of tumor sites alone reduces damage to normal tissues and organs.

The shortcomings of PDT/SDT are: sonosensitiser/photosensitizer is easily decomposed in the body, the circulation in vivo is short, the fluorescence will quench, etc. Rose Bengal (RB) has been used as a photosensitizer/sonosensitizer for a period of time and is a non-toxic and stable compound with great photosensitivity, in that the singlet oxygen quantum yield is 0.75. But the hydrophilicity of RB makes it difficult to concentrate in the target tissue, which greatly limits its application in PDT/SDT.

SUMMARY

In order to solve the problems existing in the prior art, the application provides a compound amphiphilic peptide nanomicelle, a preparation method and an application, which solve the problem of rapid elimination of the common fluorescent substance ICG, RB, MB, DOX, etc. and poor accumulation in tumor tissue, as well as in order to achieve the multifunctional application of amphiphilic peptide nanomicelles (such as bio-imaging, drug release, tumor treatment, etc.).

The technical solutions of the application comprise:
A compound amphiphilic peptide nanomicelle containing a fluorescent substance selected from the group consisting of Rose Bengal (RB), indocyanine green (ICG), methylene blue (MB), and doxorubicin (DOX).

A main body of said compound amphiphilic peptide nanomicelle is a $C_{18}$-GRRRRRRRGDS ($C_{18}GR_7RGDS$) amphiphilic peptide containing an arginine-glycine-aspartate (RGD) tripeptide sequence.

The said compound amphiphilic peptide nanomicelle has a diameter of 10 ~ 100 nanometers and a potential of -20 ~ 40 millivolts.

A preparation method of a compound amphiphilic peptide nanomicelle comprises the following steps :

a. dissolving the amphiphilic peptide $C_{18}GR_7RGDS$ in ultrapure water to prepare an amphiphilic peptide solution with a concentration of 10 g/mL;
b. dissolving Rose Bengal was dissolved in ultrapure water to prepare a solution with a concentration of 10 g/mL.
c. mixing the amphiphilic peptide solution and the fluorescence substance solution at 2:1/1:1 volume ratio followed by sonication at a frequency of 5-35 kHz, at 10-30 °C for 10-40 min with avoiding light to synthesize the compound amphiphilic peptide nanomicelle.
d. transferring the compound amphiphilic peptide nanomicelle to the dialysis bag with molecular weight cutoff of 500-1500 Dalton, to obtain an integrin $\alpha_v\beta_3$-targeted compound amphiphilic peptide nanomicelle after dialysis for 48 to 72 hours.

Use of the compound amphiphilic peptide nanomicelle in fluorescence imaging of melanoma and cervical cancer.

Use of the compound amphiphilic peptide nanomicelle in preparation of photothermal and photoacousitic agent in melanoma and cervical cancer.

Use of the compound amphiphilic peptide nanomicelle in preparation of photodynamic therapy (PDT) and sonodynamic therapy (PTT) agent in melanoma and cervical cancer.

Use of the compound amphiphilic peptide nanomicelle in preparation of a novel photodynamic therapy agent for nasopharyngeal carcinoma.

Use of the compound amphiphilic peptide nanomicelle in preparation of a novel sonodynamic therapy agent for nasopharyngeal carcinoma.

Use of the compound amphiphilic peptide nanomicelle in preparation of a novel combined therapeutic agent for photodynamic and sonodynamic therapy of nasopharyngeal carcinoma.

A compound amphiphilic peptide nanomicelle can be injected intravenously or intratumoratively, and used in a plurality of treatment methods for nasopharyngeal carcinoma.

The advantages of the application are as follows: the integrin $\alpha_v\beta_3$-targeted compound amphiphilic peptide nanomicelle and a selective construction method can develop the novel biological probe, expand the preparation technology of the fluorescent contrast agent, and improve the bioavailability of the fluorescent probe. It is of great significance to realize early molecular diagnosis, photodynamic therapy and photothermal therapy of tumor neovascularization.

The application is further introduced in combination with the figures and the specific mode of implementation below.

BRIEF DESCRIPTION OF THE DRAWINGS

Figure 1 shows a general structure and process of the targeted compound amphiphilic peptide nanomicelles above mentioned.

Figure 2 shows the stability of nanomaterials for novel targeted complex amphiphilic peptide nanomicelle fluorescent probe (Fig. 2A) and the drug loading efficacy of Rose Bengal (Fig. 2B).

Figure 3 shows the cytotoxicity of novel targeted complex amphiphilic peptide nanomicelle fluorescent probe in melanoma cells (B16) (Fig. 3A), cervical cancer cells (Hela) (Fig. 3B) and fibroblasts (L929) (Fig. 3C).

Figure 4 shows the photoacoustic effect of targeted compound amphiphilic peptide nanoparticles applied to melanoma cells (B16) (Fig. 4A) and cervical cancer cell (Hela) (Fig. 4c), respectively. And the photoacoustic effect of targeted compound amphiphilic peptide nanoparticles applied to melanoma cells (B16) (Fig. 4B) and cervical cancer cells (Hela) (Fig. 4D) at different times.

Figure 5 shows the photothermal effect and photoacoustic effect of the targeted compound amphiphilic peptide nanomicelles at different times/concentrations; The yield of ROS from PARN (peptide amphiphile-rose bengal nanomicelles) at different time periods (1, 3, 5 min) under US or Laser (Fig. 5A); The yield of ROS from PARN/RB of different RB concentrations (0, 2, 4, 6, 8, 10 $\mu$g/mL) under US (Fig. 5B); The yield of ROS from PARN/RB of different RB concentrations (0, 2, 4, 6, 8, 10 $\mu$g/mL) under Laser (Fig. 5C).

Figure 6 shows the cytotoxicity of the targeted compound amphiphilic peptide nanomicelles above mentioned in CNE-2Z cells.

Figure 7 shows the changes in the control of tumor growth from SDT and PDT in nasopharyngeal carcinoma mice after intravenous administration of compound amphiphilic peptide nanomicelles.

Figure 8 shows the changes in the control of tumor growth from SDT and PDT in nasopharyngeal carcinoma mice after intratumoral administration of compound amphiphilic peptide nanomicelles.

Figure 9 is a photograph of tumor changes in nasopharyngeal carcinoma mice from SDT and PDT during the treatment by intravenous administration of compound amphiphilic peptide nanomicelles.

Figure 10 is a photograph of tumor changes in nasopharyngeal carcinoma mice from SDT and PDT during the treatment by intratumoral administration of compound amphiphilic peptide nanomicelles.

DETAILED DESCRIPTION

The present disclosure is specifically described by the following examples, which are only used to further illustrate the present disclosure, and are not to be construed as limiting the scope of the present disclosure. The person having ordinary skill in the art may make some non-essential improvements to the present disclosure according to the contents of the above disclosure.

The raw materials used in the preparation of the disclosure are all obtained by the commodity.

The present application is specifically described by the following examples, which are only used to further illustrate the present application, and are not to be construed as limiting the scope of the present application. The person having ordinary skill in the art may make some non-essential improvements to the present application according to the contents of the above application.

The raw materials used in the preparation of the application are all obtained by the commodity.

Example 1:

Mix 2 ml of the amphiphilic peptide $C_{18}GR_7RGDS$ solution (Ningbo Kangbei Biochemical Co., Ltd., model: 817870, batch number: 17040701) (10mg/mL) and 1 mL of methylene blue solution (2 mg/mL). Then the mixed solution was placed in the ultrasonic cleaner with avoiding light, and the conditions were set at 25 °C, 30 min, and 28 kHz. After washing dialysis bag (molecular weight cutoff: 1000 Daltons) with ultrapure water, transfer the mixed solution into a dialysis bag, place a beaker containing ultrapure water dialysate and float it in the dialysate (the volume of dialysate is 2000 mL, pH=7. 4). Place the magneton in the beaker and place the beaker on the magnetic stirrer (rotation speed: 120rpm, room temperature). When the dialysis was carried out for 2 hours, the color of the dialysate was blue, the mixed solution in the dialysis bag became transparent, and the dialysis continued for 48 hours. There is no characteristic absorption peak of methylene blue after measuring the mixed solution by UV-Vis spectrophotometer, indicating that methylene blue is not encapsulated into the amphiphilic peptide nanomicelle. This also indicates that it is difficult to encapsulate the methylene blue with the amphiphilic peptide sequence synthesized by the present application to form the final product.

Example 2:

Mix 2 mL of the amphiphilic peptide $C_{18}GR_7RGDS$ solution (10 mg/mL) and 1 mL of curcumin (2 mg/mL, dissolved in acetic acid), and place the mixed solution in the ultrasonic cleaner with avoiding light(25 °C, 30 min, and 28 kHz). After washing dialysis bag (molecular weight cutoff: 1000 Daltons) with ultrapure water, transfer the mixed solution into a dialysis bag, place a beaker containing ultrapure water dialysate and float it in the dialysate (the volume of dialysate is 2.0 L, pH=7. 4). Place the magneton in the beaker and place the beaker on the magnetic agitator (rotation speed: 120 rpm, room temperature). After 3 hours of dialysis, yellow precipitates were found in dialysis bag and gradually increased with the increase of dialysis time. This was because ultrapure water enters the mixture through a dialysis bag, and curcumin was insoluble in water and precipitated. This also shows that the amphiphilic peptide sequence of the application does not encapsulate curcumin well.

Example 3:

Mix 2 mL of the amphiphilic peptide $C_{18}GR_7RGDS$ solution (Ningbo Kangbei Biochemical Co., Ltd., model: 817870, batch number: 17040701) (10 mg/mL) and 1 mL of rose bengal solution (2 mg/mL) and place the mixed solution in the ultrasonic cleaner with avoiding light (25 °C, 30 min, and 28 kHz). After washing dialysis bag (molecular weight cutoff: 1000 Daltons) with ultrapure water, transfer the mixed solution into a dialysis bag, place a beaker containing ultrapure water dialysate and float it in the dialysate (the volume of dialysate is 2000 ml, pH=7. 4). Place the magneton in the beaker and place the beaker on the magnetic stirrer (rotation speed: 120 rpm, room temperature). To replace dialysate every 4 hours and dialysis for 48 hours to get fluorescent amphiphilic peptide self-assembled nanomicelles. After testing, it was found that most of the rose bengal was successfully encapsulated into the polypeptide sequence of the application and formed self-assembled nanomicelles.

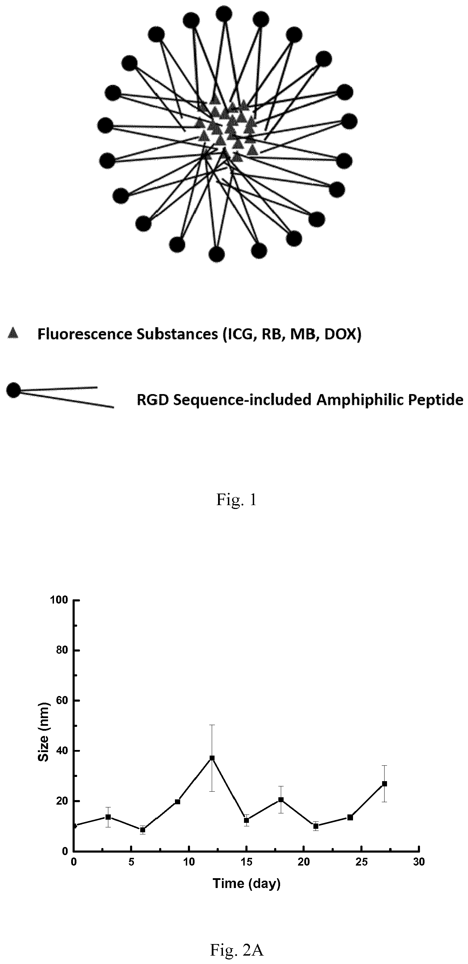

The particle size was measured by a Zetasiser every two days. It was found that the complex amphiphilic peptide was stable, and the positive charge was favorable for cell uptake (Fig. 2A).

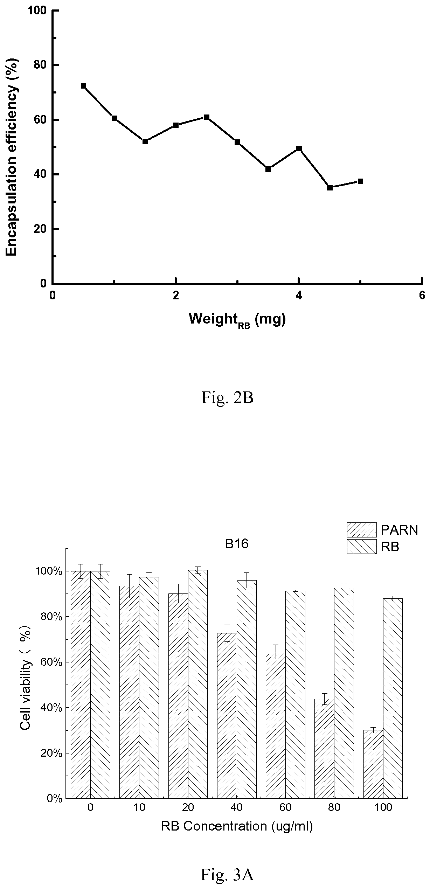

The loading ratio of rose bengal in amphiphilic peptide nanomicelles was calculated through OD value measured by ultraviolet spectrophotometer, and the loading ratio tends to be stable as the concentration of rose bengal increase (Fig. 2B).

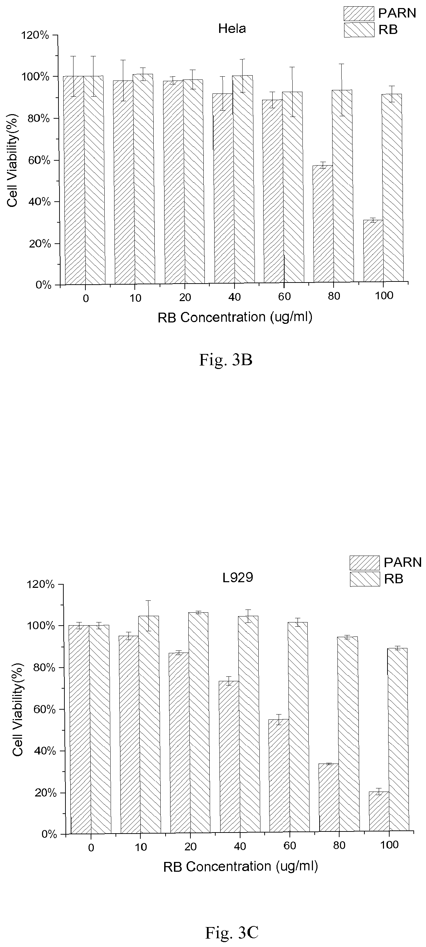

Melanoma and cervical cancer cells ($\alpha_v\beta_3^+$), and fibroblasts were plated in 96-well plates at $1 \times 10^4$ cells per well respectively, and incubated at 37 °C for 48 hours. After the cells were full, these cells were incubated with different concentrations of the compound amphiphilic peptide nanomicelles (synthesized according to the present example) and the corresponding concentrations of rose bengal (control group) separately. The different concentrations reagents used were configured by medium. After 24 hours of culture, the medium was removed, and the cell viability was measured at 450 nm with a microplate reader after treating by CCK-8. As shown in figure 3, as the concentration of rose bengal increase, the cytotoxicity of compound amphiphilic peptide nanomicelles was significantly higher than that of pure rose bengal. The compound amphiphilic peptide nanomicelles are also less toxic to normal cells such as fibroblasts. At a concentration of 20 µg/mL, the cell viability of fibroblasts is over 80%. This indicates that the compound amphiphilic peptide nanomicelles have good biocompatibility and will also use this concentration as the optimum concentration for subsequent experiments.

Example 4:

Mix 3 mL of the amphiphilic peptide $C_{18}GR_7RGDS$ solution (10 mg/mL) and 2 mL of rose bengal solution (2 mg/mL) and place the mixed solution in the ultrasonic cleaner with avoiding light (25 °C, 40 min, and 28 kHz). After washing dialysis bag (molecular weight cutoff: 1000 Daltons) with ultrapure water, transfer the mixed solution into a dialysis bag, place a beaker containing ultrapure water dialysate and float it in the dialysate (the volume of dialysate is 2000 ml, pH=7. 4). Place the magneton in the beaker and place the beaker on the magnetic stirrer (rotation speed: 120rpm, room temperature). To replace dialysate every 4 hours and dialysis for 60 hours to get fluorescent amphiphilic peptide self-assembled nanomicelles.

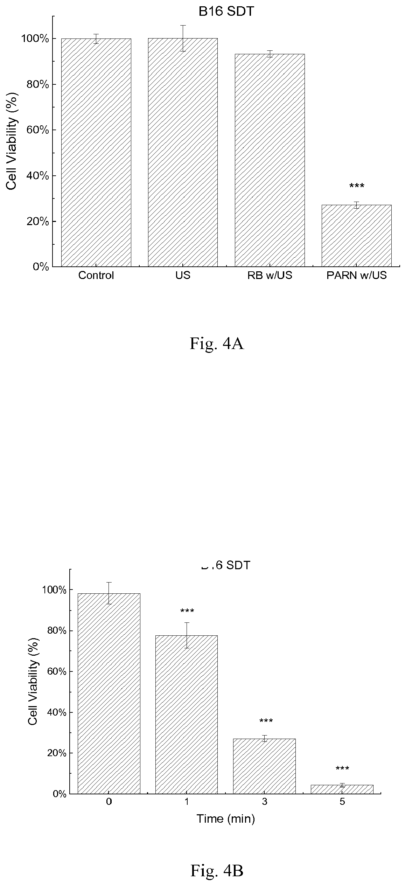

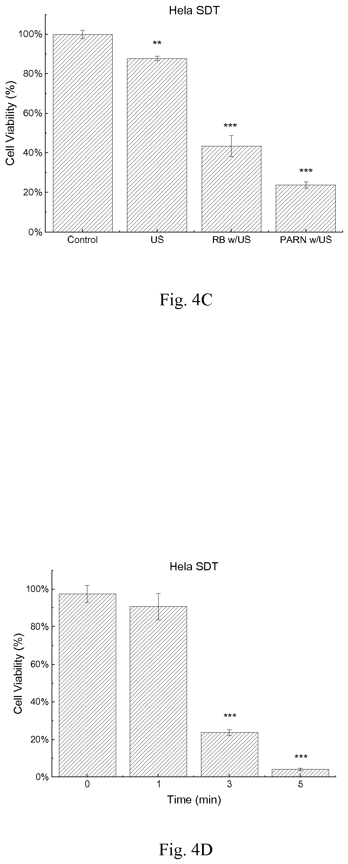

Melanoma and cervical cancer cells ($\alpha v\beta_3^+$) were plated in 96-well plates at $1 \times 10^4$ cells per well respectively, and incubated at 37°C for 48 hours. When the cells were full, 20 µg/mL of compound amphiphilic peptide nanomicelles (synthesized according to the present example) and corresponding concentrations of rose bengal (control group) were added respectively to replace the former medium. After 4 h of incubation, each well of cells was supplied with fresh medium, and subsequently treated with ultrasound/laser irradiation for 3 min. The cells were cultured for 24 hours and treated by CCK-8 method. Then the cell viability was measured at 450 nm with a microplate reader. As shown in figure 4, the photoacoustic and photothermal effects of amphiphilic peptide nanomicelles on melanoma and cervical cancer cells are illustrated.

Example 5:

Mix 1 mL of the amphiphilic peptide $C_{18}GR_7RGDS$ solution (10 mg/mL) and 1 mL of rose bengal solution (2 mg/mL) and place the mixed solution in the ultrasonic cleaner with avoiding light (25°C, 20 min, and 28 kHz). After washing dialysis bag (molecular weight cutoff: 1000 Daltons) with ultrapure water, transfer the mixed solution into a dialysis bag, place a beaker containing ultrapure water dialysate and float it in the dialysate (the volume of dialysate is 2000 ml, pH=7. 4). Place the magneton in the beaker and place the beaker on the magnetic stirrer (rotation speed: 120 rpm, room temperature). After 72 hours dialysis, fluorescent amphiphilic peptide self-assembled nanomicelles were obtained.

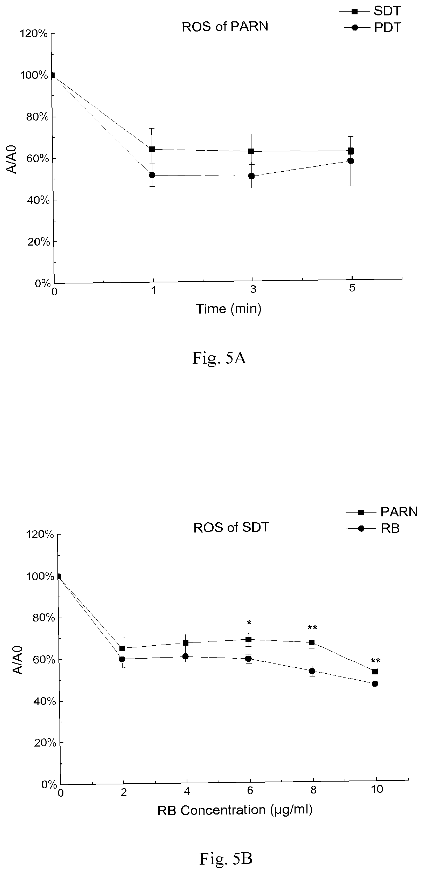

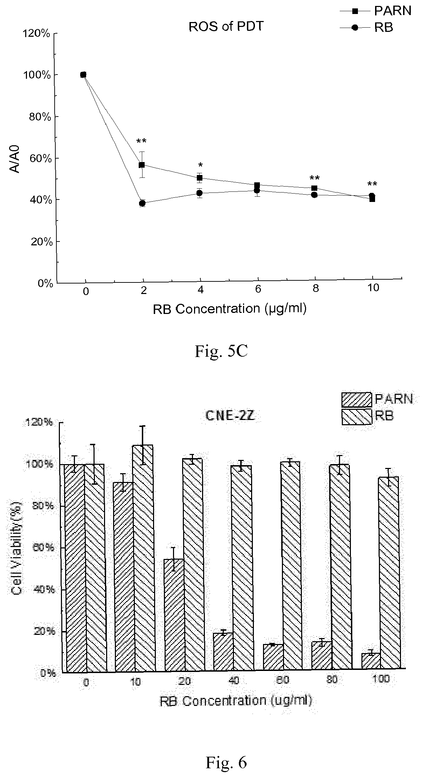

2 mL compound amphiphilic peptide self-assembled nanomicelles and rose bengal solutions with rose bengal concentrations (20 $\mu$g/mL) were prepared and mixed with 20 $\mu$L 1,3-diphenyl isobenzofuran (DPBF) (2 mg/mL in acetonitrile), respectively. The OD values were measured at 548 nm with a microplate reader. Then the mixed solution was treated by laser (808 nm, 1.5 W/cm$^2$, 0/1/3/5min) or ultrasound (1 W/cm$^2$, 0/1/3/5min) and measured the OD value at 548 nm again. Results as shown in figure 5, the OD value of the compound nanomicelles decreased with the increase of time, and 3 minutes was selected as the optimal treatment time for the follow-up experiment.

2 mL different concentrations compound amphiphilic peptide self-assembled nanomicelles and corresponding concentrations of rose bengal were prepared and mixed with 20 $\mu$L DPBF, respectively. After measuring the OD values of mixed solution at 548 nm with a microplate reader, the mixed solution was treated by laser (808 nm, 1.5 w/cm$^2$, 3 min) or ultrasound (1 W/cm$^2$, 3 min) and measured the OD value at 548 nm again. As a result (Fig. 5), the absorption peak at 548 nm of DPBF is reduced after oxidation, which can be used to quantify the reactive oxygen species (ROS) produced by photosensitizers and sonosensitizers after laser irradiation or ultrasound. It is concluded that the compound nanomicelle produces more active oxygen.

Figure 1 shows a general structure and process of the compound amphiphilic peptide nanomicelles synthesized according to present application. Figure 2 shows the stability of nanomaterials and the change in the loading rate of Rose Bengal. As shown in figure 2, the material has good stability, with no significant change in particle size within 30 days ranging from 10 to 40 nanometers, which makes it easier for nanoscale substances to enter the cell through the cell membrane. The loading rate of rose bengal tends to saturation with the increase of the quality of rose bengal. The prepared amphiphilic peptide nanoparticles have good biocompatibility and safety (figure 3). The compound amphiphilic peptide nanomicelles have good sonodynamic and photodynamc effects in melanoma and cervical cancer cells (figure 4), and the compound amphiphilic peptide nanomicelles have perfect photodynamic and sonodynamic (figure 5).

The compound amphiphilic peptide targeting melanoma and cervical cancer integrin $\alpha v\beta_3$ as a fluorescent imaging, SDT and PDT material can not only enrich the ways of non-invasive (or minimally invasive) treatment of melanoma and cervical cancer. Moreover it is of great significance for the development of new cancer diagnosis and treatment mode, reducing the side effects of current clinical treatment, reducing the damage to normal tissues, and improving the efficacy and accuracy of cancer diagnosis. At the same time, it has important practical value for promoting the clinical transformation of the amphiphilic peptide as a novel melanoma and cervical cancer targeted diagnosis and treatment.

Example 6:

First of all, the designed concentration (0, 20, 40, 60, 80 and 100 $\mu$g/mL) solution of nanomicelle and rose bengal were prepared with 1640 medium. CNE-2Z cells were seeded into a 96-well player at a density of $2 \times 10^4$ cells per well. Each sample with a certain concentration was added to 5 wells. The cytotoxicity of the pure rose bengal and the nanomicelle solution at different concentrations was compared. After culturing for 48 hours, the cells were grown to 80%, and the medium containing different volumes of nanomicelle and rose bengal solution was added instead of the original medium according to the experimental group setting. After an additional incubation for 24 h at 37°C in the dark, fresh medium (100 $\mu$L) together with CCK-8 (10 $\mu$L) was added followed by fluorescence analysis on a microplate reader at a wavelength of 450 nm. Cell viability was calculated according to the formula, with the maximum and minimum of OD values excluded:

$$\text{Cell Viability} = \text{average of } (OD\text{-}OD_{blk})/\text{average of } (OD_0 - OD_{blk}) \times 100\%$$

As shown in figure 6, the pure rose bengal is less toxic to CNE-2Z cells and has no rising tendency with the increase of concentration. However, the nanomicelles we synthesized are more toxic to NE-2Z cells, and with the increase of concentration, the cell viability is lower and lower, 20 micrograms per milliliter, the survival rate has been reduced to 50%. The nanomicelles we synthesized are more toxic to CNE-2Z cells, and with the increase of concentration, the cell survival rate is lower and lower. And the survival rate has dropped to 50% at 20 $\mu$g/mL.

Example 7:

20 nude mice bearing CNE-2Z tumors were randomly divided into five groups: control group, untreated group (PARN), PDT group (PARN+ Laser), SDT group (PARN+ US), combination therapy group (PARN+ Laser+ US).Then 200 $\mu$L of PARN was injected into mice via the tail vein except control group. For PDT group, the tumors of mice were treated by the 808 nm laser at 1.5 W/cm$^2$ for 3 min after 5 h after vein administration of PARN, the distance between tumor and light source is 2 cm. As to SDT group, the tumors of mice were conducted by US in the pre-set condition (frequency: 3 MHz, power density: 3 W/ cm$^2$, duty cycle: 50%, time: 3 min). For combination therapy group, ultrasound treatment after laser irradiation was acted on tumor, the treatment conditions are the same as above. The groups of intratumoral injection treatment were similar to the intravenous groups. In addition to the control group, 150 $\mu$L of PARN was injected into tumor tissue in each group, and the laser or ultrasound was applied to the tumor site instantly, the treatment conditions are the same as those described above.

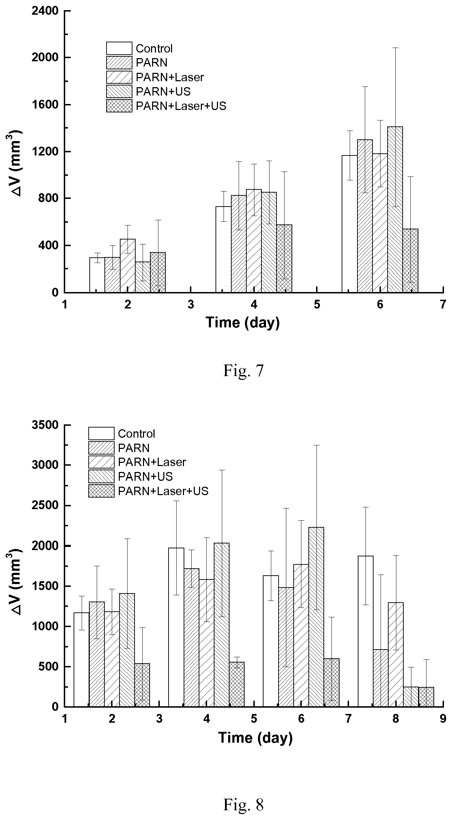

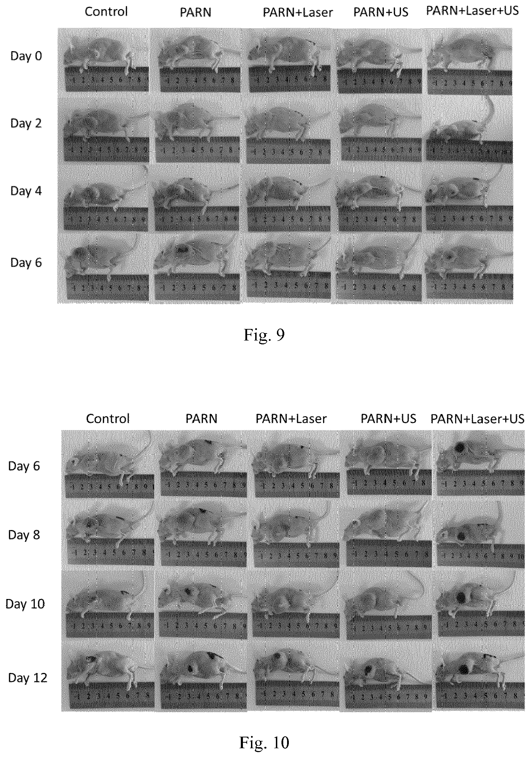

As shown in figure 7 and figure 9, the combined treatment group (PARN+ Laser+ US) had a significant inhibitory effect on CNE-2Z tumors after intravenous injection. It was observed that the growth of the tumor was significantly inhibited in PARN+ Laser, PARN+ US and PARN+ Laser+ US when PARN was administrated by intratumoral injection.

The present application has been described using specific examples to explain the principles and embodiments of the present application. The description of the above examples is only for helping to understand the method and the central idea of the present application. It should be noted that those skilled in the art can make various improvement and modifications to the present application without departing from the theory of the application, and these improvement and modifications also fall within the protection of the claims of the present application.

**Claims**

1. A compound amphiphilic peptide nanomicelle containing a fluorescent substance selected from the group consisting of indocyanine green (ICG), Rose Bengal (RB), methylene blue (MB), and doxorubicin (DOX).

2. The compound amphiphilic peptide nanomicelle of claim 1, wherein a main body of said compound amphiphilic peptide nanomicelle is a $C_{18}$-GRRRRRRRRGDS ($C_{18}GR_7RGDS$) amphiphilic peptide containing an arginine-glycine-aspartate (RGD) tripeptide sequence.

3. The compound amphiphilic peptide nanomicelle of claim 2, wherein the compound amphiphilic peptide nanomicelle has a diameter of 10 to 100 nm and a potential of -20 to 40 mV.

4. A method for preparing the compound amphiphilic peptide nanomicelle according to any one of claim 1, comprising following steps:

   a. dissolving the amphiphilic peptide $C_{18}GR_7RGDS$ in ultrapure water to prepare an amphiphilic peptide solution with a concentration of 10 g/mL;
   b. dissolving the fluorescence substance in ultrapure water to prepare a fluorescence substance solution with a concentration of 10 g/mL;
   c. these above-mentioned mixing the amphiphilic peptide solution and the fluorescence substance solution at 2:1 or 1:1 volume ratios followed by sonication at a frequency of 5-35 kHz, at 10-30 °C for 10-40 min with avoiding light to synthesize the compound amphiphilic peptide nanomicelle; and
   d. transferring the compound amphiphilic peptide nanomicelle to a dialysis bag with a molecular weight cutoff of 500-1500 Dalton to obtain an integrin $\alpha_v\beta_3$-targeted compound amphiphilic peptide nanomicelle targeted after dialysis for 48-72 h.

5. The method of claim 4, wherein the fluorescence substance is Rose Bengal.

6. Use of the compound amphiphilic peptide nanomicelle of claim 1 for preparation of photothermal and photoacousitic agent or photodynamic therapy (PDT) and sonodynamic therapy (PTT) agent in melanoma and cervical cancer.

7. The compound amphiphilic peptide nanomicelle of claim 1, wherein said compound amphiphilic peptide nanomicelle is a new photodynamic therapy agent for nasopharyngeal carcinoma.

8. The compound amphiphilic peptide nanomicelle of claim 1, wherein said compound amphiphilic peptide nanomicelle is a new sonodynamic therapy agent for nasopharyngeal carcinoma.

9. The compound amphiphilic peptide nanomicelle of claim 1, wherein said compound amphiphilic peptide nanomicelle is a new combined therapeutic agent for photodynamic and sonodynamic therapy for nasopharyngeal carcinoma.

10. The compound amphiphilic peptide nanomicelle of claim 1, wherein said compound amphiphilic peptide nanomicelle is injected intravenously or intratumoratively, and used in a plurality of treatment methods for nasopharyngeal carcinoma.

11. The method of claim 4, wherein the dialysis bag in step d has a molecular weight cutoff of 500-1000 Dalton.

▲ Fluorescence Substances (ICG, RB, MB, DOX)

● RGD Sequence-included Amphiphilic Peptide

Fig. 1

Fig. 2A

Fig. 2B

Fig. 3A

Fig. 3B

Fig. 3C

Fig. 4A

Fig. 4B

Fig. 4C

Fig. 4D

Fig. 5A

Fig. 5B

Fig. 5C

Fig. 6

Fig. 7

Fig. 8

Fig. 9

Fig. 10

Europäisches
Patentamt

European
Patent Office

Office européen
des brevets

**EUROPEAN SEARCH REPORT**

Application Number

EP 19 17 6088

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/007664 A1 (UNIV NORTHEASTERN [US]) 14 January 2016 (2016-01-14) * examples 1-3 * * claims 1,14,24 * | 1-6,11 | INV. A61K41/00 A61K47/64 A61K47/69 A61P35/00 C07K7/00 |
| X | HASSANZADEH F ET AL: "Novel NGR anchored pullulan micelles for controlled and targeted delivery of doxorubicin to HeLa cancerous cells", IRANIAN POLYMER JOURNAL, IRAN POLYMER AND PETROCHEMICAL INSTITUTE, IR, vol. 27, no. 4, 14 February 2018 (2018-02-14), pages 263-274, XP036464198, ISSN: 1026-1265, DOI: 10.1007/S13726-018-0606-9 [retrieved on 2018-02-14] * abstract * * scheme 3 * * table 1 * | 1,6 | |
| X | GUO X D ET AL: "Core/shell pH-sensitive micelles self-assembled from cholesterol conjugated oligopeptides for anticancer drug delivery", AICHE JOURNAL, vol. 56, no. 7, 18 July 2010 (2010-07-18), pages 1922-1931, XP55119564, ISSN: 0001-1541, DOI: 10.1002/aic.12119 * abstract * * figure 1 * | 1 | TECHNICAL FIELDS SEARCHED (IPC) A61K C07K |
| X | CN 103 301 482 B (SHENZHEN INST OF ADV TECH CAS) 17 December 2014 (2014-12-17) * abstract * * examples 4-11 * * figure 5 * | 1 | |

-/--

The present search report has been drawn up for all claims

| Place of search | Date of completion of the search | Examiner |
|---|---|---|
| The Hague | 2 December 2019 | Bliem, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 19 17 6088

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 108 948 152 A (SHANGHAI INST MATERIA MEDICA CAS) 7 December 2018 (2018-12-07) * paragraphs [0038] - [0040] * * figures 1-6 * ----- | 1 | |

TECHNICAL FIELDS
SEARCHED       (IPC)

The present search report has been drawn up for all claims

| Place of search | Date of completion of the search | Examiner |
|---|---|---|
| The Hague | 2 December 2019 | Bliem, Barbara |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons
 
& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 19 17 6088

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2019

| Patent document cited in search report | | Publication date | Patent family member(s) | | Publication date |
|---|---|---|---|---|---|
| WO 2016007664 | A1 | 14-01-2016 | CA 2954545 A1 | | 14-01-2016 |
| | | | EP 3166643 A1 | | 17-05-2017 |
| | | | JP 2017525676 A | | 07-09-2017 |
| | | | US 2017202783 A1 | | 20-07-2017 |
| | | | WO 2016007664 A1 | | 14-01-2016 |
| CN 103301482 | B | 17-12-2014 | NONE | | |
| CN 108948152 | A | 07-12-2018 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910034439
- CN 201910047595